# EUROPÄISCHE PATENTSCHRIFT

(11) **EP 1 442 031 B1**
(45) Veröffentlichungstag und Bekanntmachung des Hinweises auf die Patenterteilung: **09.03.2011**
(21) Anmeldenummer: 02772026.7
(22) Anmeldetag: 22.08.2002
(51) Int. Cl.: C07D 295/02, C07D 251/54, C07D 251/56, C07C 277/00, C09K 21/04, C08K 5/49, C08K 3/32

(54) **VERFAHREN ZUR HERSTELLUNG VON POLYPHOSPHATEN ORGANISCHER BASEN**
METHOD FOR PRODUCING POLYPHOSPHATES OF ORGANIC BASES
PROCEDE DE PRODUCTION DE POLYPHOSPHATES DE BASES ORGANIQUES

(30) Priorität: 13.09.2001 DE 10145093
(43) Veröffentlichungstag der Anmeldung: 04.08.2004
(73) Patentinhaber: Chemische Fabrik Budenheim KG, 55253 Budenheim (DE)
(72) Erfinder: GÖTZMANN, Karl, 55257 Budenheim (DE); NÄGERL, Hans-Dieter, 67373 Dudenhofen (DE); FUTTERER, Thomas, 65197 Wiesbaden (DE)
(74) Vertreter: Weber, Roland
(86) Internationale Anmeldenummer: PCT/DE2002/003065
(87) Internationale Veröffentlichungsnummer: WO 2003/024945

(56) Entgegenhaltungen:
- EP-A- 0 049 763
- EP-A- 0 849 260
- WO-A-97/44377
- US-A- 6 114 421

## Beschreibung

Polyphosphate organischer Stickstoffbasen, wie von Guanidin und Melamin, bekommen zunehmende Bedeutung als Flammschutzmittel in Kunststoffen und als Flammfestausrüstung von Textilien. Für Kunststoffe als Flammschutzmittel verwendete Polyphosphatgemische sollen sich möglichst nahe dem Zersetzungspunkt des Kunststoffes zersetzen, da zu niedrige Zersetzungspunkte bereits bei der Herstellung des Gemisches von Kunststoff und Flammschutzmittel zur Zersetzung und damit zur Wasserdampfbildung und unerwünschten Bläschenbildung im Kunststoffprodukt führen. Um solche unerwünscht niedrigen Zersetzungspunkte des Flammschutzmittels zu vermeiden, sollen die Polyphosphatgemische möglichst kein Orthophosphat und ggf. auch kein Pyrophosphat enthalten.

Bisher sind verschiedene Verfahrenstypen zur Herstellung von Polyphosphaten organischer Basen bekannt, doch haben diese bekannten Verfahren alle bestimmte Nachteile.

Eine Verfahrenstype zur Herstellung von Polyphosphaten organischer Basen besteht darin, zunächst ein Orthophosphat der organischen Base herzustellen und dieses dann durch Erhitzen auf eine Temperatur über 300°C in Polyphosphate der organischen Base umzuwandeln. Dieses Verfahren ist beispielsweise in der WO 97/44 377 beschrieben. Die erforderlichen relativ hohen Temperaturen dieser Verfahrenstype machen das Verfahren unwirtschaftlich, wenn man insbesondere bedenkt, daß es sich bei Flammschutzmitteln um Massenprodukte handelt. Gemäß der US 6 114 421 werden bestimmte organische Stickstoffbasen in bestimmten Konzentrationen und bei bestimmten Reaktionsbedingungen mit Phosphorsäureanhydrid umgesetzt.

Eine andere Verfahrenstype besteht darin, handelsübliche Polyphosphorsäure mit der organischen Base zu neutralisieren. Handelsübliche Polyphosphorsäuren sind jedoch Mischungen, die Ortho- und Pyrophosphorsäure enthalten, so daß die Produktgemische das unerwünschte Ortho- und Pyrophosphat der organischen Base enthalten. Außerdem sind die handelsüblichen Polyphosphorsäuren zähflüssig bis fest und schwer zu verarbeiten. Die enthaltenen Ortho- und Pyrophosphate werden erst bei Temperaturen oberhalb 306°C in Polyphosphate umgewandelt, was den gleichen Nachteil wie die obenbeschriebene erste Verfahrenstype ergibt.

Eine dritte Verfahrenstype geht von Alkalipolyphosphat oder Alkalipyrophosphat aus, die in Wasser gelöst und mit HCl angesäuert werden. Die Produkte müssen zur Beseitigung der Chloridionen ausgewaschen und getrocknet werden, was das Verfahren aufwendig und umständlich macht. Chloridionen dürfen bei der Verwendung als Flammschutzmittel nicht in dem Flammschutzmittel enthalten sein, da sie zu schädlichen Nebenprodukten führen können.

Die EP-A-0 849 260 offenbart ein Verfahren zur Herstellung von Flammschutzmitteln, wobei man ein Gemisch von Phosphorpentoxid, wenigstens einer organischen Stickstoffbase und Wasser umsetzt. Das molare Verhältnis von Wasser zu Phosphorpentoxid liegt im Bereich von 1 bis 3, und für die Zuführung des Wassers zu der Reaktion werden drei Varianten offenbart, nämlich als freies Wasser, als Hydratwasser der organischen Stickstoffbase, zum Beispiel von hydratisiertem Piperazin, oder als Hydratwasser von hydratisiertem Salz, wie beispielsweise Trinatriumphosphat oder Borax.

Die der Erfindung zugrundeliegende Aufgabe bestand darin, unter Vermeidung der Nachteile des Standes der Technik ein einfaches und kostengünstiges Verfahren zur Herstellung von Polyphosphaten organischer Basen zu bekommen, wobei die Produkte möglichst frei von Orthophosphaten und ggf. auch von Pyrophosphaten sein sollen.

Gelöst wird diese Aufgabe durch ein Verfahren zur Herstellung von Polyphosphaten organischer Basen, bei dem man ein Gemisch von Phosphorpentoxid und wenigstens einer organischen Stickstoffbase mit wenigstens einer Verbindung, die bei den Reaktionsbedingungen unter Zersetzung Wasser abgibt und außer Wasser nur flüchtige Zersetzungsprodukte bildet, in solchem Molverhältnis umsetzt, daß bei der Zersetzung der wasserabgebenden Verbindung je Mol Phosphorpentoxid höchstens im wesentlichen 2 Mol Wasser entstehen, wobei die wasserabgebende Verbindung unter wasserfreier Oxalsäure, Oxalsäure-Dihydrat und Ameisensäure ausgewählt ist.

Polyphosphate, im Sinne der Erfindung, sind kondensierte Phosphate mit einer Kettenlänge von mindestens 2.

Überraschenderweise wird damit die Schwierigkeit des Standes der Technik, die nötige Wassermenge homogen und gleichmäßig in dem Gemisch von Phosphorpentoxid und Base zu verteilen, gelöst. Die erforderliche Menge an Wasser wird absolut homogen und gleichmäßig in der gewöhnlich vorgelegten Mischung von Phosphorpentoxid und organischer Stickstoffbase verteilt, so daß örtliches Überangebot an Wasser, das zur Bildung von Ortho- und Pyrophosphorsäure führt, vermieden wird.

Des weiteren wird die der Erfindung zugrunde liegende Aufgabe durch ein Gemisch von nach dem erfindungsgemäßen Verfahren hergestellten Polyphosphaten organischer Stickstoffbasen gelöst, die a) einen Gewichtsverlust beim Erhitzen auf 320°C von weniger als 2 Gew.-%, b) einen pH-Wert einer 10 gew.-%-igen wäßrigen Aufschlämmung bei 25°C von >5, vorzugsweise von 5,2 bis 7,7, besonders von 5,8 bis 7,0, und c) eine Löslichkeit in Wasser bei 25°C von weniger als 0,1, vorzugsweise weniger als 0,01 g/100 ml Wasser haben.

Erfindungsgemäß verwendet man als wasserabgebende Verbindungen solche, die außer Wasser nur flüchtige Zersetzungsprodukte ergeben, nämlich Oxalsäure in wasserfreier Form oder als Dihydrat oder Ameisensäure, die bei der Zersetzung außer Wasser nur Kohlendioxid und Kohlenmonoxid ergeben, welche aufgrund ihrer Flüchtigkeit das Reaktionsgemisch verlassen.

Die Molverhältnisse in dem Reaktionsgemisch sind entsprechend den obigen Ausführungen so einzustellen, daß je Mol P₂O₅ im Reaktionsgemisch höchstens etwa 2 Mol Wasser entstehen. Dabei hat man zu berücksichtigen, daß 1 Mol Oxalsäure (wasserfrei) bei der Zersetzung 1 Mol Wasser ergibt und Oxalsäuredihydrat dabei 3 Mol Wasser ergibt. Bei der Herstellung von Pyrophosphat verwendet man die Ausgangsstoffe in solchem Molverhältnis, daß im wesentlichen 2 Mol Wasser je Mol P₂O₅, an der wasserabgebenden Verbindung entstehen. Bei der Herstellung langkettiger Polyphosphate liegt dieser Wert bei etwa 1 Mol Wasser je Mol P₂O₅. Wenn in dieser Beschreibung von "etwa" oder "im wesentlichen" die Rede ist, liegt der Wert gewöhnlich bei ± 10, vorzugsweise ± 5 %des angegebenen Wertes.

Als organische Stickstoffbase kann jede für diesen Zweck an sich bekannte Stickstoffbase verwendet werden, wie beispielsweise Polyvinylamin, Polyethylenimin, Piperazin, Methylendiamin, Melamin, Guanidin, Methylolmelamin oder deren Kondensate sowie deren Gemische. Melamin und Guanidin sind dabei bevorzugt. Bei Annahme, daß die Stickstoffbasen einen Basenrest besitzen, ist das Molverhältnis so einzustellen, daß bei erwünschter Gewinnung von Pyrophosphat und vollständiger Absättigung des Pyrophosphates mit Basenresten je P - Atom im Produkt im wesentlichen 2 Mol Base eingesetzt werden müssen. Bei beabsichtigter Gewinnung langkettiger Polyphosphate soll das verwendete Molverhältnis von Base: P im wesentlichen 1 :1 sein (Base : P₂O₅ im wesentlichen 2: 1). Durch Einstellung des Molverhältnisses kann die Absättigung mit Baseresten variiert werden.

Die erfindungsgemäße Umsetzung kann bei Umgebungstemperatur erfolgen, doch ist sie dabei für praktische Zwecke im Regelfall zu langsam. Daher ist es bevorzugt, die Umsetzung bei erhöhter Temperatur vorzunehmen, insbesondere im Bereich von 100 bis 250 °C, vorzugsweise 180 bis 250 °C, besonders bevorzugt 200 bis 220 °C. Die bevorzugte Mindesttemperatur von 200 °C liegt über der Schmelztemperatur der Oxalsäure, was sich auf die Reaktion der Komponenten positiv auswirkt. Andererseits liegen die anzuwendenden Temperaturen erheblich unter den bei bekannten Verfahren erforderlichen Temperaturen von über 300 °C. Dies macht das Verfahren wirtschaftlich, wobei zu berücksichtigen ist, daß durch die homogene Verteilung des Wassers im Reaktionsgemisch die Entstehung von Orthophosphat und ggf. Pyrophosphat vermieden wird.

Vorzugsweise geht man beim efindungsgemäßen Verfahren so vor, daß man die erforderliche Menge an Phosphorpentoxid mit der organischen Stickstoffbase vermischt, das Gemisch auf die gewünschte Temperatur bringt und dann die erforderliche Menge der wasserabgebenden Verbindung zugibt und durch Vermischen homogen in dem Reaktionsgemisch verteilt.

Die erfindungsgemäßen Polyphosphatgemische werden mit Vorteil als Flammschutzmittel für Kunststoffe, vorzugsweise Thermoplaste, insbesondere Polyamide und Polyester verwendet.

Die nachfolgenden Beispiele dienen der weiteren Erläuterung der Erfindung

### Beispiel 1

Ein ölbeheizter Doppel-Z-Kneter mit einem Nutzvolumen von 5 l wird mit 1250 g (10 Mol) Melamin und 710 g (5 Mol) Phosphorpentoxid beschickt. Die Mischung wird homogenisiert und auf eine Temperatur von 220 °C erhitzt.

Innerhalb von 15 min werden dann 450 g (5 Mol) wasserfreie Oxalsäure zugegeben und untergemischt. Nach der Zugabe erfolgt noch eine Nachreaktionszeit von 15 min, ehe das entstandene Melaminpolyphosphat aus dem Kneter entnommen wird.

### Beispiel 2

In dem gleichen Kneter, wie in Beispiel 1 beschrieben, werden 1260 g (10 Mol) Melamin und 710 g (5 Mol) Phosphorpentoxid gemischt und auf 220 °C aufgeheizt. Wie bei Beispiel 1 werden innerhalb von 15 min jedoch 900 g (10 Mol) wasserfreie Oxalsäure eingebracht. Nach weiteren 15 min bei 220 °C wird dem Kneter reines Dimelaminpyrophosphat entnommen.

### Beispiel 3

In einem Labor-Pflugscharmischer mit einem Nutzinhalt von 10 l und thermoölbeheiztem Doppelmantel werden 3780 g Melamin (30 Mol) mit 2130 g (15 Mol) Phosphorpentoxid vermischt und auf eine Temperatur von 200 °C erhitzt. Innerhalb von 30 min werden dann 630 g (5 Mol) Oxalsäuredihydrat zugegeben. Nach einer Nachreaktionszeit von weiteren 15 min wurde das gebildete Melaminpolyphosphat aus dem Mischer entnommen.

### Beispiel 4

In dem gleichen Mischer, wie in Beispiel 3 beschrieben, werden 5400 g (30 Mol) Guanidincarbonat mit 2130 g (15 Mol) Phosphorpentoxid vermischt und auf eine Temperatur von 250 °C aufgeheizt. Über einen Zeitraum von 1 h werden 1350 g (15 Mol) wasserfreie Oxalsäure zudosiert. Nach einer weiteren Stunde Nachreaktionszeit wird das entstandene Guanidinpolyphosphat aus dem Mischer entnommen.

### Beispiel 5

In einem Kneter gemäß Beispielen 1 und 2 werden 861 g (10 Mol) Piperazin mit 710 g (5 Mol) Phosphorpentoxid gemischt und auf 100 °C aufgeheizt. Innerhalb von 15 min werden dann 210 g (1,7 Mol) Oxalsäuredihydrat zugegeben. Die Nachreaktionszeit beträgt 1 h, ehe das entstandene Piperazinpolyphosphat entnommen wird.

## Patentansprüche

1. Verfahren zur Herstellung von Polyphosphaten organischer Basen, bei dem man ein Gemisch von Phosphorpentoxid und wenigstens einer organischen Stickstoffbase mit wenigstens einer Verbindung, die bei den Reaktionsbedingungen unter Zersetzung Wasser abgibt und außer Wasser nur flüchtige Zersetzungsprodukte bildet, in solchem Molverhältnis umsetzt, daß bei der Zersetzung der wasserabgebenden Verbindung je Mol Phosphorpentoxid höchstens im wesentlichen 2 Mol Wasser entstehen, wobei die wasserabgebende Verbindung unter wasserfreier Oxalsäure, Oxalsäure-Dihydrat und Ameisensäure ausgewählt ist.

2. Verfahren nach Anspruch 1, **dadurch gekennzeichnet, daß** man als organische Stickstoffbase Melamin und/oder Guanidin und/oder Piperazin, vorzugsweise Melamin verwendet.

3. Verfahren nach einem der Ansprüche 1 und 2, **dadurch gekennzeichnet, daß** man die Umsetzung bei einer Temperatur im Bereich von 100 bis 250 °C, vorzugsweise 180 bis 250 °C, besonders 200 bis 220 °C durchführt.

4. Gemisch von Polyphosphaten organischer Stickstoffbasen, hergestellt nach einem der Ansprüche 1 bis 3, **gekennzeichnet durch** a) einen Gewichtsverlust beim Erhitzen auf 320°C von weniger als 2 Gew.-%, b) einen pH-Wert einer 10 gew.-%-igen wäßrigen Aufschlämmung bei 25°C von >5, vorzugsweise von 5,2 bis 7,7, besonders von 5,8 bis 7,0, und c) eine Löslichkeit in Wasser bei 25°C von weniger als 0,1, vorzugsweise weniger als 0,01 g/100 ml Wasser.

5. Verwendung eines Gemisches von Polyphosphaten nach Anspruch 4 als Flammschutzmittel für Kunststoffe, vorzugsweise Thermoplaste, insbesondere Polyamide und Polyester.

## Claims

1. Process for the preparation of polyphosphates of organic bases, wherein a mixture of phosphorus pentoxide and at least one organic nitrogen base is reacted with at least one compound which releases water and in addition to water, forms only volatile decomposition products accompanied by decomposition under the reaction conditions in such a molar ratio that upon decomposition of the water-releasing compound, at most essentially 2 mol. water are produced per mol. phosphorus pentoxide, wherein the water-releasing compound is selected from an hydrous oxalic acid, oxalic acid dehydrate and formic acid.

2. Process according to claim 1, **characterized in that** melamine and/or guanidine and/or piperazine, preferably melamine is used as organic nitrogen base.

3. Process according to one of claims 1 and 2, **characterized in that** the reaction is carried out at a temperature in the range from 100 to 250°C, preferably 180 to 250°C, particularly 200 to 220°C.

4. Mixture of polyphosphates of organic nitrogen bases prepared according to one of claims 1 to 3, **characterized by** a) a weight loss upon heating to 320°C of less than 2 wt.-%, b) a pH value of a 10 wt.-% aqueous suspension at 25°C of > 5, preferably from 5.2 to 7.7, particularly from 5.8 to 7.0 and c) a solubility in water at 25°C of less than 0.1, preferably less than 0.01 g/100 ml water.

5. Use of a mixture of polyphosphates according to claim 4 as flame-protection agents for plastics, preferably thermoplasts, in particular polyamides and polyesters.

## Revendications

1. Procédé de fabrication de polyphosphates de bases organiques, selon lequel un mélange de pentoxyde de phosphore et d'au moins une base azotée organique est mis en réaction avec au moins un composé qui, dans des conditions de réaction, libère de l'eau en se décomposant et génère, à l'exception de l'eau, uniquement des produits de décomposition volatils, dans un rapport molaire tel que, pendant la décomposition du composé libérant de l'eau, un maximum de sensiblement 2 moles d'eau par mole de pentoxyde de phosphore soit produit, le composé libérant de l'eau étant choisi parmi l'acide oxalique anhydre, l'acide oxalique dihydraté et l'acide formique.

2. Procédé selon la revendication 1, **caractérisé en ce que** l'on utilise comme base azotée organique de la mélamine et/ou de la guanidine et/ou de la pipérazine, de préférence de la mélamine.

3. Procédé selon l'une des revendications 1 et 2, **caractérisé en ce que** la réaction est mise en oeuvre à une température comprise dans la plage allant de 100 à 250 °C, de préférence de 180 à 250 °C, en particulier de 200 à 220 °C.

4. Mélange de polyphosphates de bases azotées organiques, fabriqué selon l'une des revendications 1 à 3, **caractérisé par** a) une perte pondérale lors du chauffage à 320 °C de moins de 2 % en poids, b) une valeur de pH d'une suspension aqueuse à 10 % en poids, à une température de 25 °C, supérieure à 5 , de préférence de 5,2 à 7,7, en particulier de 5,8 à 7,0, et c) une solubilité dans l'eau à 25 °C de moins de 0,1, de préférence de moins de 0,01 g/100 ml d'eau.

5. Utilisation d'un mélange de polyphosphates selon la revendication 4 en tant qu'agent ignifuge pour plastiques, de préférence thermoplastiques, en particulier polyamides et polyesters.
